# EUROPEAN PATENT SPECIFICATION

(11) **EP 2 609 861 B1**
(45) Date of publication and mention of the grant of the patent: **19.12.2018**
(21) Application number: 12199663.1
(22) Date of filing: 28.12.2012
(51) Int. Cl.: A61B 6/03, A61B 6/04, A61B 6/14, A61B 6/00

(54) **Apparatus for acquiring panoramic, teleradiographic and optionally volumetric CBCT radiographies**
Vorrichtung zur Erfassung von DVT-Panorama-, Teleradiologie- und optionale Volumen-Röntgenaufnahmen
Appareil d'acquisition panoramique, téléradiographique et éventuellement radiographies CBCT volumétriques

(30) Priority: 28.12.2011 IT BO20110764
(43) Date of publication of application: 03.07.2013
(73) Proprietor: CEFLA S.C., 40026 Imola (IT)
(72) Inventor: Bianconi, Davide, 40023 Castel Guelfo di Bologna (IT); Guardini, Luca, 37047 San Bonifacio (IT); Righini, Dario, 40026 Imola (IT)
(74) Representative: Herrmann, Franz

(56) References cited:
- WO-A1-98/20796
- US-A- 3 673 408
- US-A- 5 500 884
- US-A1- 2010 034 340
- US-A1- 2011 150 185

## Description

The present invention relates to the technical field of extraoral dental radiography, and particularly to an apparatus alternatively performing panoramic radiographies, cranial teleradiographies and optionally cone beam volumetric radiographies of facial skeleton. All these types of radiographies are well known in the art.

Panoramic radiography (also known as orthopantomography) produces a radiographic image of a curved plan approximating patient jaws, with blurring of the anatomical structures lying outside a narrow layer around the predesigned curved plane. In the following, this kind of acquisition is also indicated with PAN.

Teleradiography is a projective radiographic technique, producing radiographic images of the skull or of other anatomical areas from different projections, with minimum magnification and geometrical distortion. Usually two perspectives are represented, latero-lateral and antero-posterior.

Cone beam volumetric radiography (also known as CBCT) is the acquisition, from different projection angles, of a series of two-dimensional radiographic images which will be processed post-acquisition to reconstruct three-dimensional volumes.

It is well known in the art that the positions of PAN, CBCT and teleradiographic sensors, and therefore the distance of the specific sensor from the X-ray source, are appropriately chosen so as to get the best radiographic result. The distance between X-ray source and X-ray sensor varies according to the kind of sensor. Generally speaking, the distance between teleradiographic sensor and X-ray source is the largest, while the distance between PAN sensor and X-ray source is smaller than the distance between CBCT sensor and X-ray source.

For instance, the distances, which allow getting optimal radiographic results, are the following:
- X-ray source - PAN sensor distance: 520-580 mm;
- X-ray source - CBCT sensor distance: 600-700 mm;
- X-ray source - teleradiographic sensor distance: 1400-1600 mm.

Radiographic apparatuses acquiring panoramic and teleradiographic radiographies have been on the market for over 50 years. This kind of apparatuses generally has a C-arc, at whose ends the X-ray source and the X-ray detector are positioned at an appropriate distance to acquire panoramic images.

When a teleradiographic acquisition has to be performed, the X-ray detector must be positioned at a greater distance from the X-ray source than allowed by the dimension of the C-arc. For this reason, such apparatuses are provided with a supplementary arm supporting the X-ray detector at an appropriate distance for performing teleradiography.

Traditionally, such apparatuses are provided with an X-ray source and at least one X-ray detector. When only one X-ray detector is present, the X-ray detector must be manually or automatically relocated in the desired position for panoramic or teleradiographic acquisition. When more than one X-ray detector is present, means for removing obstacles along the X-ray path between the X-ray source and X-ray detector for the specific acquisition are also present. One of the many examples of such apparatuses is disclosed in patent application WO 2007/018332 A1.

This kind of apparatus shows many drawbacks.

The first drawback lies in the high cost of X-ray detectors, which nowadays cost four to ten times the price of an X-ray source.

The second drawback, tightly linked to the first, lies in the fact that in the apparatuses provided with one X-ray detector only, which has to be manually relocated to perform the desired acquisition, there is a great risk of breakage, in that the X-ray detector is delicate and can undergo dangerous accidental falls during relocation. Moreover, in the apparatus removable connection systems must be provided, which must be safe under the point of view of electric transmission and reliable in the repeatability of positioning.

The third drawback lies in the fact that, due to acquisition geometry, on the same apparatus two different suitable patient positioning systems must be present, one for PAN/CBCT and one for teleradiography, with ensuing cost increase.

When during the same session a panoramic and a teleradiographic acquisition must be performed on the same patient (for instance an orthodontic patient), first the panoramic acquisition must be performed, then: in the simpler case of two X-ray detectors, the patient has to be moved from the first to the second patient positioning system; in the more complicate case of one X-ray detector only, the patient must be moved from the first to the second patient positioning system and the X-ray detector must be relocated. This leads to a significant prolonging of acquisition time for both operator and patient.

US 2010/0034340 A1 discloses an apparatus for performing panoramic and cephalographic imaging with an X-ray sensor and an X-ray source, wherein the raw images of cephalographic imaging are processed to remove distortion and differences in magnification.

US 5 500 884 A discloses an apparatus for panoramic imaging which is combined with an X-ray source and an intraoral detector for intraoral X-ray imaging.

Proceeding from this related art, the present invention seeks to provide an apparatus of economic construction for acquiring panoramic, teleradiographic and optionally CBCT volumetric images, having a simplified design and improved operations for both operator and patient.

This object is achieved by the apparatus and the method having the features of the independent claim. Advantageous embodiments and refinements are specified in claims dependent thereon.

According to the present invention, there is provided an apparatus having two X-ray sources, each positioned at an appropriate distance from an X-ray sensor, so that good quality images can be obtained. The X-ray detector can be the same for the three (panoramic, teleradiographic, volumetric) kind of acquisitions.

In order to perform the desired (panoramic, teleradiographic or CBCT volumetric) acquisition, suitable means must be provided for removing the obstacles (X-ray sensor, collimator or X-ray source) from the X-ray path of X-ray radiation emitted by the X-ray source used for the specific acquisition.

The X-ray detector can be a two-dimensional area sensor, preferably a digital X-ray detector comprising a two-dimensional array of detector elements or an one-dimensional linear sensor, preferably a digital X-ray detector comprising at least a row of detector elements. In the case of an area sensor, which is the simplest and most suitable solution, teleradiography can be performed in one-shot mode, reducing emission time, patient dose and image processing. In this case it is sufficient to align the second X-ray source to the area X-ray sensor, removing the first X-ray source from the second source X-ray path.

In the case of a linear sensor, different methods are possible to perform teleradiography with or without the movement of the X-ray source during acquisition:
1) In a first method, the X-ray source remains essentially stationary during acquisition. The movement of the linear sensor is synchronous both with the movement of a secondary collimator, located in the X-ray path between X-ray source and X-ray detector or between X-ray source and patient, and with a suitable primary collimator, located in the vicinity of the X-ray source and ensuring the irradiation of the X-ray sensor during the entire acquisition.
2) In a second method, the X-ray source is suitably roto-translated to complete the acquisition. The movement of the linear sensor is synchronous with a secondary collimator integral with the X-ray source. This method does not include a relative movement of the primary collimator with respect to the X-ray source during acquisition.

In both cases, the final image is the result of a post-acquisition processing combining the plurality of linear images.

In the present invention, when a linear sensor is to be used, both methods 1) and 2) can be used; suitable means must be present in the apparatus to remove obstacles from the X-ray path and for moving the second X-ray source in case 2).

Further advantages and properties of the present invention are disclosed in the following description, in which exemplary embodiments of the present invention are explained in detail based on the drawings:
- Figure 1: shows a perspective schematic view of a prior art radiographic apparatus as a whole;
- Figure 2a: is a front view of the present invention apparatus as a whole;
- Figure 2b: is a top view of the present invention apparatus as a whole.

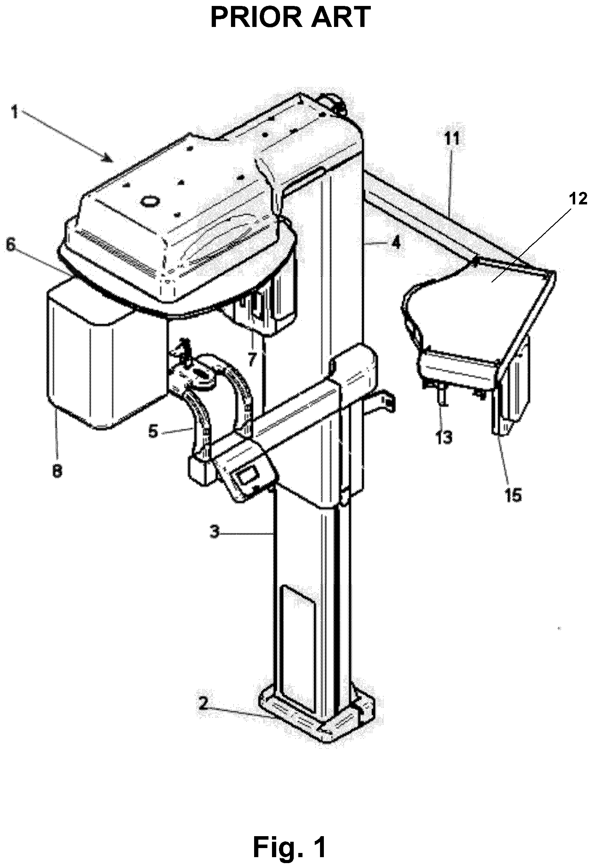

Figure 1 shows in its entirety a prior art radiographic apparatus 1 for performing alternatively panoramic, teleradiographic and optionally also CBCT volumetric acquisitions. Apparatus 1 comprises a base 2, a post 3 supporting an extension 4, which is provided with a vertical section sliding on the post 3 and a horizontal section. The vertical section of the extension 4 allows the vertical movement of a C-arm 6, which is attached to the horizontal section of the extension 4, and which in its turn supports an X-ray source 7, and an X-ray detector 8. In a more complete version of the apparatus, an alternating mechanism (not shown) is present, allowing to alternate a PAN sensor and a CBCT sensor. Moreover, a device 5 for the positioning of the patient is present.

On a further arm 11, arranged on extension 4, a support 12 for a teleradiographic sensor 15 for teleradiography and a further device 13 for positioning the patient are present.

In all acquisition modalities, the X-rays must impinge on the specific sensor: for the specific radiographic acquisition the sensor must be brought in the position allowing that the X-ray radiation impinges on the detector.

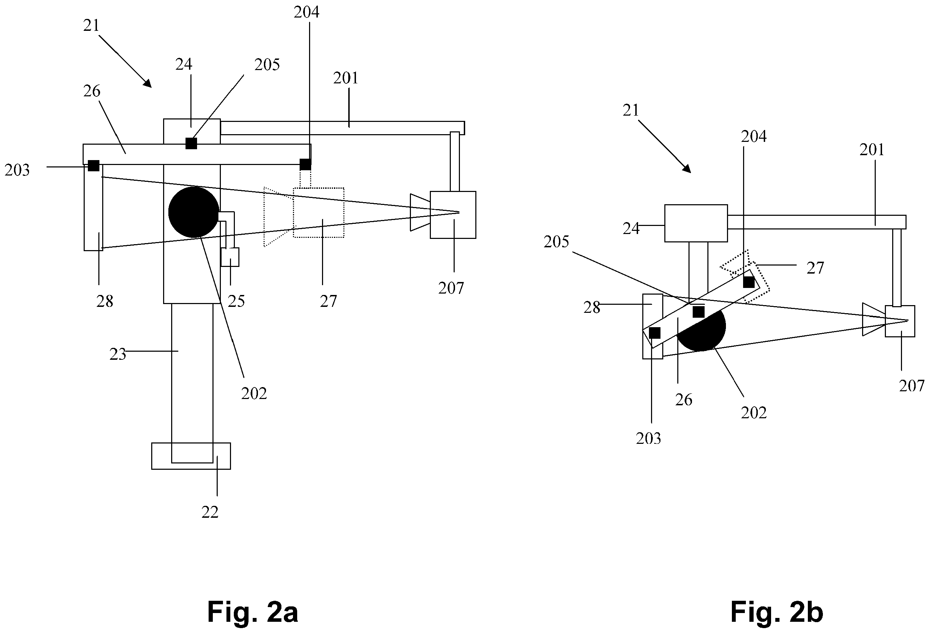

In Figures 2, showing the present invention, the elements numbered 21 to 28 correspond to the elements numbered from 1 to 8 in Figure 1, while the elements numbered 201 to 207 represent predominantly additional elements according to the present invention with respect to Figure 1.

Figure 2a shows in its entirety an apparatus 21 for alternatively performing panoramic, teleradiographic and optionally CBCT volumetric acquisitions.

Apparatus 21 comprises a base 22, a post 23 supporting an extension 24, which is provided with a vertical section sliding on the post 23 and a horizontal section. The vertical section of the extension 24 allows the vertical movement of a C-arm 26, which is attached to the horizontal section of the extension 24, and which in its turn supports a first X-ray source 27, and an X-ray detector 28. C-arm 26 is a rotary arm rotating around a patient skull 202, represented in a stylized form, during the acquisition of panoramic and CBCT volumetric images.

In a different apparatus configuration, performing CBCT volumetric acquisitions in addition to panoramic acquisitions, means have to be provided, which allow to respect the optimal distance between X-ray source 27 and X-ray sensor 28 during the respective acquisition. In the preferred embodiment wherein only one X-ray sensor 28 is present, means are provided, that allow adjusting the distance between X-ray source 27 and X-ray detector 28.

In a different embodiment wherein two X-ray sensors are present, i.e. one PAN and one CBCT volumetric X-ray sensor, there is provided also an alternating mechanism allowing alternating the PAN and CBCT sensor. The alternating mechanism allows to bring the PAN and CBCT sensor at the required distance with respect to the X-ray source. For instance the PAN and CBCT sensor may each be mounted on a common holder, which allows moving alternatively the PAN sensor or the CBCT sensor into the X-ray path. For instance, the holder might be rotatable around a pivotation axis. Pivoting the holder around the pivotation axis may then bring either the PAN or CBCT sensor into the X-ray path.

Moreover, only one single patient positioning device 25 is present. The positioning device 25 allows fixing the position and/or orientation of the patient skull 202 during the whole acquisition process independent of the acquisition mode. The positioning device 25 may be a bite, on which the patient can bite thus fixing the dentition of the patient. Instead of the bite or in addition to the bite, the positioning device may comprise support elements. These support elements may be in contact with the outside of the head of the patient. For example the support elements may be in contact with the nasion or with both sides of the head, for instance in contact with the orifice of one or both ears. In most cases the positioning device will be rigidly attached to the extension 24 or the post 23, allowing an adjustment to the size of the patient, but keeping the patient stationary during the acquisition process.

The apparatus 21 further comprises a second X-ray source 207, which is mounted on an arm 201 at a distance from patient 202 appropriately chosen to perform a cranial teleradiography. In the embodiment shown in the Figures 2 the second X-ray source 207 is mounted on an arm 201 fixed on extension 24, but in other embodiments the arm 201 might be fixed to other portion of the apparatus 21 or even to a wall.

Moreover, in Figure 2a means 203 for moving X-ray detector 28 are shown. Said means 203 for moving X-ray sensor 28 can be used for:
1) adjustment of the spatial relationship between X-ray sensor 28 and first X-ray source 27, in particular the distance between X-ray sensor 28 and first X-ray source 27, during panoramic or volumetric acquisition;
2) alignment of the X-ray sensor 28 with the second X-ray source 207 during teleradiographic acquisition;
3) movement of the X-ray sensor 28 during teleradiographic acquisition if a one-dimensional, linear sensor is used for the X-ray sensor 28.

The apparatus 21 may also be provided with means 204 for moving the first X-ray source 27, which can be used for:
1) adjustment of the spatial relationship between X-ray sensor 28 and first X-ray source 27, in particular the distance between X-ray sensor 28 and first X-ray source 27, during panoramic or volumetric acquisition;
2) removal of the first X-ray source 27 from the X-ray path of the X-ray radiation emitted by the second X-ray source 207 during teleradiographic acquisition.

The apparatus 21 can also be provided with means 205 for moving the C-arm 26 to impart an particular angular orientation and spatial position to the C-arm 26 itself, allowing to remove obstacles from the X-ray path of the X-ray radiation emitted by the second X-ray source 207 during teleradiography. The means 205 may include a drive for pivoting or rotating the C-arm 26 around a vertically aligned rotation axis and may further comprise a linear xy-drive, which allows the rotation axis to be linearly shifted in a direction at right angle to the rotation axis.

Moving means 203, 204, 205 can each be present alone, or can be combined in any way depending on the requirements of the imaging modes that can be performed by the apparatus 21.

Figure 2b shows a top view on the apparatus 21 of Figure 2a, in the preferred configuration for performing a cranial teleradiography of a patient skull 202.

To perform a cranial teleradiography, the first X-ray source 27 must be removed from the X-ray path of X-ray radiation emitted by the second X-ray source 207. The configuration shown in Figure 2b is preferred, wherein the X-ray detector 28 is an area sensor and therefore teleradiography can be acquired in standard mode (one-shot). Using different X-ray detectors is still possible, and consequently the configuration of the apparatus 21 is to be modified for acquiring a cranial teleradiography.

In the preferred embodiment, wherein only one single area sensor is present, which allows to acquire panoramic, teleradiographic and optionally volumetric images, said area sensor is configured for the acquisition of panoramic images such that only a portion of the sensor area is sensitive to X-rays (PAN mode). Such portion corresponds to the area effectively hit by the X-ray beam emitted by the first X-ray source 27 during PAN acquisition.

One of the advantages of the present invention lies in the possibility of using only one single X-ray sensor appropriately chosen for all the three (panoramic, teleradiographic and CBCT volumetric) types of acquisition without the need to relocate it to another holder for teleradiography, therefore freeing the apparatus from the risk of damages during X-ray relocation. Such simplification allows building a particularly cheap machine due to the presence of only one single sensor and the maximal simplicity of teleradiographic acquisition.

Another advantage of the present acquisition lies in the possibility of performing on the same patient during the same session both panoramic (or optionally volumetric) and teleradiographic acquisitions, without the need to relocate or to change the orientation of the patient in order to set up the apparatus. With the present apparatus, the patient is positioned and oriented only once on the only patient positioning system 25, and panoramic (and optionally volumetric) and teleradiographic acquisition can be performed successively, only positioning the X-ray source 27 and 207 and X-ray detector 28 needed for the desired acquisition.

From the economic point of view, a further advantage of the present invention lies in the possibility of using different technologies for the X-ray sources 27 and 207, using a rotating anode X-ray tube as the first X-ray source 27, more complex and expensive but with better performances especially for CBCT volumetric acquisitions, and a simpler and cheaper fixed anode X-ray tube as the second X-ray source 207 for acquiring cranial teleradiographies.

Throughout the description and claims of this description, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment or example of the invention are to be understood to be applicable to any other aspect, embodiment or example described herein unless incompatible therewith.

### Reference Numerals:

- 1: apparatus
- 2: base
- 3: post
- 4: extension
- 5: patient positioning system
- 6: C-arm
- 7: X-ray source
- 8: X-ray detector
- 11: arm for teleradiography support
- 13: teleradiographic patient positioning system
- 15: teleradiographic sensor
- 21: new apparatus
- 22: base
- 23: post
- 24: extension
- 25: patient positioning system
- 26: C-arm
- 27: first X-ray source
- 28: X-ray sensor
- 201: arm for the second X-ray source
- 202: patient skull
- 203: means for moving X-ray sensor 28
- 204: means for moving first X-ray source 27
- 205: means for moving C-arm 26
- 207: second X-ray source

## Claims

1. Apparatus for acquiring panoramic, teleradiographic and optionally CBCT volumetric dental radiographies, comprising a support holding a rotary arm (26) which in its turn supports at one end a first X-ray source (27) and at the other end an X-ray detector (28) at a suitable distance for acquiring panoramic and optionally CBCT volumetric images,
wherein
- the apparatus comprises only one single patient positioning system (25) for the acquisition of panoramic, teleradiographic and optionally CBCT volumetric dental radiographies,
- the apparatus comprises a second X-ray source (207) at a distance from the detector (28) suitable for acquiring cranial teleradiographies, and wherein
- the apparatus comprises means (203, 204, 205) for removing the first X-ray source (27) from the X-ray path of the X-ray radiation emitted by the second X-ray source (207) during teleradiographic acquisition.

2. Apparatus according to claim 1,
wherein the apparatus comprises only one X-ray detector (28).

3. Apparatus according to claim 1 or 2,
wherein the X-ray detector (28) is an area sensor.

4. Apparatus according to claim 3,
wherein the area sensor is used in a PAN mode for panoramic acquisitions and in standard mode for teleradiographic and optionally CBCT volumetric acquisitions.

5. Apparatus according to claim 1 or 2,
wherein the X-ray detector is a linear sensor.

6. Apparatus according to claim 1,
wherein two X-ray detectors are present including one first sensor for acquiring panoramic and teleradiographic images and a second sensor for acquiring CBCT volumetric images, both sensors mounted on a mechanism allowing to alternate them.

7. Apparatus according to any one of the preceding claims, wherein the first X-ray source (27) is a rotating anode X-ray tube and/or wherein the second X-ray source (207) is a fixed anode X-ray tube.

8. Apparatus according to any one of the preceding claims, wherein the patient positioning system (25) is arranged for keeping the dentition of the patient continuously fixed at the same position and orientation for the acquisition of panoramic, teleradiographic and optionally CBCT volumetric dental radiographies.

9. Apparatus according to any one of the preceding claims, wherein the apparatus comprises means (203, 204, 205) for locating the X-ray detector (28) and/or the first X-ray source (27) at a first relative position with respect to the rotary arm (26) for the acquisition of panoramic radiographies, and at second relative position with respect to the rotary arm (26) for the acquisition of teleradiographic radiographies.

10. Apparatus according to any one of the preceding claims, wherein the apparatus further comprises means (203, 204) for adjusting the distance between the first X-ray source (27) and the X-ray detector (28) to the acquisition mode.

11. Apparatus according to any one of the preceding claims, wherein the apparatus comprises means (203) for alignment of the X-ray detector (28) with the second X-ray source (207) during teleradiographic acquisition.

12. Apparatus according to any one of the preceding claims, wherein the apparatus further comprises one or more devices chosen from the group consisting of: means (203) for moving X-ray detector (28), means (204) for moving the first X-ray source (27), means (205) for moving C-shaped rotary arm (26), such means having the aim of removing obstacles from the X-ray path between the second X-ray source (207) and X-ray detector (28).

13. Method for sequentially performing panoramic and teleradiographic and optionally CBCT volumetric acquisitions, using the apparatus according to any one of claims 1 to 11, comprising the following steps:
- positioning the patient in the only patient positioning system (25), which is used for acquiring panoramic, teleradiographic and optionally CBCT volumetric dental radiographies;
- acquiring panoramic or optionally CBCT volumetric images using the first X-ray source (27) and the X-ray detector (28) ;
- removing the first X-ray source (27) from the X-ray path of the X-ray radiation emitted by the second X-ray source (207); and
- acquiring teleradiographic images using the second X-ray source (207) and the X-ray detector (28).

## Patentansprüche

1. Vorrichtung zur Aufnahme von dentalen panoramischen, teleradiographischen und wahlweise volumetrischen CBCT-Radiographien mit einer Halterung, die einen Dreharm (26) hält, der seinerseits an einem Ende eine erste Röntgenquelle (27) hält und an dem anderen Ende einen Röntgendetektor (28) in einem geeigneten Abstand zur Aufnahme von panoramischen und wahlweise volumetrischen CBCT-Bildern hält,
wobei
- die Vorrichtung nur ein einzelnes Patientenpositioniersystem für die Aufnahme von dentalen panoramischen, teleradiographischen und wahlweise volumetrischen CBCT-Radiographien aufweist,
- die Vorrichtung eine zweite Röntgenquelle (207) in einem Abstand vom Detektor (28) aufweist, der für die Aufnahme von kranialen Teleradiographien geeignet ist, und wobei
- die Vorrichtung Mittel (203, 204, 205) aufweist, um die erste Röntgenquelle (27) aus dem Röntgenstrahlweg der Röntgenstrahlung zu entfernen, die von der zweiten Röntgenquelle (207) während teleradiographischen Aufnahmen emittiert wird.

2. Vorrichtung nach Anspruch 1,
bei der die Vorrichtung lediglich einen Röntgendetektor (28) aufweist.

3. Vorrichtung nach Anspruch 1 oder 2,
bei der der Röntgendetektor (28) ein Flächensensor ist.

4. Vorrichtung nach Anspruch 3,
bei der der Flächensensor in einem PAN-Modus für panoramische Aufnahmen und in einem Standardmodus für teleradiographische und wahlweise volumetrische CBCT-Aufnahmen verwendet wird.

5. Vorrichtung nach Anspruch 1 oder 2,
bei der der Röntgendetektor ein linearer Sensor ist.

6. Vorrichtung nach Anspruch 1,
bei der zwei Röntgendetektoren vorhanden sind, einschließlich einem ersten Sensor für die Aufnahme von panoramischen und teleradiographischen Bildern und einen zweiten Sensor für die Aufnahme von volumetrischen CBCT-Bildern, wobei beide Sensoren an einem Mechanismus angebracht sind, der es gestattet, diese abwechselnd anzuwenden.

7. Vorrichtung nach einem der vorhergehenden Ansprüche,
bei der die erste Röntgenquelle (27) eine Röntgenröhre mit Drehanode und/oder bei der die zweite Röntgenquelle (207) eine Röntgenröhre mit fester Anode ist.

8. Vorrichtung nach einem der vorhergehenden Ansprüche,
bei der das Patientenpositioniersystem (25) dazu eingerichtet ist, das Gebiss des Patienten kontinuierlich in der gleichen Position und Ausrichtung für die Aufnahme von dentalen panoramischen, teleradiographischen und wahlweise volumetrischen CBCT-Radiographien festzuhalten.

9. Vorrichtung nach einem der vorhergehenden Ansprüche,
bei der die Vorrichtung Mittel (203, 204, 205) aufweist, um den Röntgendetektor (28) und/oder die erste Röntgenquelle (27) in einer ersten relativen Position bezüglich des Dreharms (26) für die Aufnahme von panoramischen Radiographien und in einer zweiten relativen Position bezüglich des Dreharms (26) für die Aufnahme von teleradiographischen Radiographien anzuordnen.

10. Vorrichtung nach einem der vorhergehenden Ansprüche,
bei der die Vorrichtung ferner Mittel (203, 204) aufweist, um den Abstand zwischen der ersten Röntgenquelle (27) und dem Röntgendetektor (28) auf den Aufnahmemodus einzustellen.

11. Vorrichtung nach einem der vorhergehenden Ansprüche,
bei der die Vorrichtung Mittel (203) umfasst, um den Röntgendetektor (28) während der teleradiographischen Aufnahmen auf die zweite Röntgenquelle (207) auszurichten.

12. Vorrichtung nach einem der vorhergehenden Ansprüche,
bei der die Vorrichtung ferner ein oder mehrere Geräte aufweist, die aus der Gruppe ausgewählt sind, die besteht aus: Mitteln (203) zum Bewegen des Röntgendetektors (28), Mitteln (204) zum Bewegen der ersten Röntgenquelle (27), Mitteln (205) zum Bewegen des C-förmigen Dreharms (26), wobei diese Mittel zum Ziel haben, Hindernisse aus dem Röntgenstrahlungsweg zwischen der zweiten Röntgenquelle (207) und dem Röntgendetektor (28) zu entfernen.

13. Verfahren um eine Folge von panoramischen, teleradiographischen und wahlweise volumetrischen CBCT-Aufnahmen unter Verwendung der Vorrichtung nach einem der Ansprüche 1 bis 11 durchzuführen, mit den folgenden Schritten:
- Positionieren des Patienten in dem einzigen Patientenpositioniersystem (25), das für die Aufnahme von dentalen panoramischen teleradiographischen und wahlweise volumetrischen CBCT-Radiographien verwendet wird;
- Aufnehmen von panoramischen oder wahlweise volumetrischen CBCT-Bildern unter Verwendung der ersten Röntgenquelle (27) und des Röntgendetektors (28);
- Entfernen der ersten Röntgenquelle (27) aus dem Röntgenstrahlungsweg der Röntgenstrahlung, die von der zweiten Röntgenquelle (207) emittiert wird;
- Aufnehmen von teleradiographischen Bildern unter Verwendung der zweiten Röntgenquelle (207) und dem Röntgendetektor (28) .

## Revendications

1. Dispositif permettant d'obtenir des radiographies dentaires panoramiques, téléradiographiques et optionnellement volumétriques CBCT, comprenant un support maintenant un bras rotatif (26) qui soutient à son tour à une extrémité une première source de rayons X (27) et à l'autre extrémité un détecteur de rayons X (28) à une distance appropriée pour acquérir des images panoramiques et optionnellement volumétriques CBCT,
dans lequel
- le dispositif comprend un seul système de positionnement du patient (25) pour obtenir des radiographies dentaires panoramiques, téléradiographiques et optionnellement volumétriques CBCT,
- le dispositif comprend une deuxième source de rayons X (207) à une distance du détecteur (28) appropriée pour obtenir des téléradiographies crâniennes, et dans lequel
- le dispositif comprend les moyens (203, 204, 205) de retirer la première source de rayons X (27) du trajet de rayons X du rayonnement X émis par la deuxième source de rayons X (207) pendant l'acquisition téléradiographique.

2. Dispositif selon la revendication 1,
dans lequel le dispositif comprend un seul détecteur de rayons X (28).

3. Dispositif selon la revendication 1 ou 2,
dans lequel le détecteur de rayons X (28) est un capteur de surface.

4. Dispositif selon la revendication 3,
dans lequel le capteur de surface est utilisé en mode PAN pour les acquisitions panoramiques et en mode standard pour les acquisitions téléradiographiques et optionnellement volumétriques CBCT.

5. Dispositif selon la revendication 1 ou 2,
dans lequel le détecteur de rayons X est un capteur linéaire.

6. Dispositif selon la revendication 1,
dans lequel deux détecteurs de rayons X sont présents y compris un premier capteur permettant d'acquérir des images panoramiques et téléradiographiques et un second capteur permettant d'acquérir des images volumétriques CBCT, les deux capteurs étant montés sur un mécanisme permettant de les alterner.

7. Dispositif selon l'une quelconque des revendications précédentes,
dans lequel la première source de rayons X (27) est un tube à rayons X à anode tournante et/ou dans lequel la deuxième source de rayons X (207) est un tube à rayons X à anode fixe.

8. Dispositif selon l'une quelconque des revendications précédentes,
dans lequel le système de positionnement du patient (25) est agencé de manière à garder la dentition du patient continuellement fixée dans la même position et la même orientation pour l'acquisition des radiographies dentaires panoramiques, téléradiographiques et optionnellement volumétriques CBCT.

9. Dispositif selon l'une quelconque des revendications précédentes,
dans lequel le dispositif comprend les moyens (203, 204, 205) de placer le détecteur de rayons X (28) et/ou la première source de rayons X (27) à une première position relative par rapport au bras rotatif (26) pour l'acquisition de radiographies panoramiques, et à une deuxième position relative par rapport au bras rotatif (26) pour l'acquisition de radiographies téléradiographiques.

10. Dispositif selon l'une quelconque des revendications précédentes,
dans lequel le dispositif comprend également les moyens (203, 204) d'ajuster la distance entre la première source de rayons X (27) et le détecteur de rayons X (28) au mode d'acquisition.

11. Dispositif selon l'une quelconque des revendications précédentes,
dans lequel le dispositif comprend les moyens (203) d'aligner le détecteur de rayons X (28) avec la deuxième source de rayons X (207) pendant l'acquisition téléradiographique.

12. Dispositif selon l'une quelconque des revendications précédentes,
dans lequel le dispositif comprend également un ou plusieurs dispositifs choisis parmi le groupe consistant en: des moyens (203) de déplacer le détecteur de rayons X (28), des moyens (204) de déplacer la première source de rayons X (27), des moyens (205) de déplacer le bras rotatif (26) en forme de C, ces moyens ayant pour but de retirer les obstacles du trajet des rayons X entre la deuxième source de rayons X (207) et le détecteur de rayons X (28).

13. Méthode pour effectuer des acquisitions panoramiques et téléradiographiques et optionnellement volumétriques CBCT de manière séquentielle, à l'aide du dispositif selon une des revendications 1 à 11, comprenant les étapes suivantes:
- positionnement du patient dans le dispositif de positionnement du patient (25), utilisé pour obtenir des radiographies dentaires panoramiques, téléradiographiques et optionnellement volumétriques CBCT;
- acquérir des images panoramiques ou optionnellement volumétriques CBCT en utilisant la première source de rayons X (27) et le détecteur de rayons X (28);
- retirer la première source de rayons X (27) du trajet des rayons émis par la deuxième source de rayons X (207);
- acquérir des images téléradiographiques en utilisant la deuxième source de rayons X (207) et le détecteur de rayons X (28) .
